# FASCICULE DE BREVET EUROPEEN

(11) **EP 1 727 501 B1**
(45) Date de publication et mention de la délivrance du brevet: **24.06.2009**
(21) Numéro de dépôt: 05729307.8
(22) Date de dépôt: 18.02.2005
(51) Int. Cl.: A61F 5/00

(54) **Ballon intra-gastrique à poches avec un organe d'obturation assemblé de façon améliorée sur lesdites poches**
Mit Beutel versehener intragastricher Ballon mit einem Verschlusselement, das auf verbesserte Weise an den Beuteln angeordnet ist
Pouch-equipped intragastric balloon comprising a sealing member which is assembled to the pouches in an improved manner

(30) Priorité: 20.02.2004 FR 0401730
(43) Date de publication de la demande: 06.12.2006
(73) Titulaire: Compagnie Européenne d'Etude et de Recherche de Dispositifs pour l'Implantation par Laparoscopie, 38200 Vienne (FR)
(72) Inventeur: PAGANON, Pascal, F-69360 Serezin du Rhône (FR); RICOL, Jean-Paul, Gilbert, 69210 Saint Germain sur L'Arbresle (FR)
(74) Mandataire: Martin, Didier Roland Valéry
(86) Numéro de dépôt international: PCT/FR2005/000388
(87) Numéro de publication internationale: WO 2005/089685

(56) Documents cités:
- DE-A- 2 822 925
- DE-A- 3 326 061
- FR-A- 2 834 202
- FR-A- 2 862 525
- US-A- 4 315 509
- US-A- 4 739 758

## Description

### DOMAINE TECHNIQUE

La présente invention se rapporte au domaine technique général des dispositifs implantables dans le corps humain destinés à être utilisés dans le cadre d'un traitement de l'obésité, et notamment de l'obésité morbide, et tout particulièrement à des implants aptes à réduire artificiellement le volume de l'estomac, en vue notamment de produire une sensation de satiété chez le patient.

La présente invention se rapporte à un ballon intra-gastrique expansible, destiné à être implanté au sein de l'estomac d'un patient dans le cadre d'un traitement de l'obésité, et comportant :
- une première poche, suffisamment souple pour passer d'une configuration à volume réduit à une configuration expansée, et pourvue d'au moins un orifice,
- une deuxième poche, disposée de manière à contenir la première poche, et pourvue d'au moins une lumière,
- un organe d'obturation, fixé de façon étanche sur la deuxième poche et destiné à obturer ledit orifice et ladite lumière.

Un tel ballon intra-gastrique expansible est par exemple décrit dans le document FR-283 4202-A1.

L'invention concerne également un procédé de fabrication d'un ballon intra-gastrique expansible destiné à être implanté au sein de l'estomac d'un patient dans le cadre d'un traitement de l'obésité, dans lequel on prévoit :
- une étape de fabrication d'au moins une première poche, suffisamment souple pour passer d'une configuration à volume réduit à une configuration expansée, et pourvue d'au moins un orifice,
- une étape d'assemblage de la première poche avec une deuxième poche, pourvue d'au moins une lumière, de telle sorte que la première poche soit contenue dans la deuxième poche,
- une étape de fabrication d'un organe d'obturation destiné à obturer ledit orifice et ladite lumière,
- une étape de fixation de l'organe d'obturation sur la deuxième poche de façon sensiblement étanche.

### TECHNIQUE ANTERIEURE

Les ballons intra-gastriques utilisés pour le traitement de l'obésité comportent généralement une ou plusieurs poche(s) souple(s) concentriques, réalisée(s) à partir d'un même matériau, tel que du silicone, et apte(s) à passer d'une configuration à volume réduit ou repliée à une configuration expansée conférant au ballon sa forme fonctionnelle.

Les poches sont ainsi souvent remplies avec un fluide de gonflage, et comportent à cet effet un organe d'obturation, du genre valve, destiné à assurer leur étanchéité. A cet effet, les poches sont munies d'un orifice destiné à être obturé par l'organe d'obturation, ce dernier étant généralement fabriqué dans le même matériau que les poches et soudé ou collé avec le pourtour de l'orifice.

La conception de ces ballons intra-gastriques, si elle présente certains avantages, notamment en matière de simplicité et d'économie de fabrication, souffre néanmoins de plusieurs inconvénients, liés en particulier à la technique d'assemblage des composants (poches et organe d'obturation) par soudure ou collage.

Ainsi, s'il est relativement facile de souder ou coller entre eux des matériaux de même nature, il peut s'avérer plus délicat de réaliser cette opération pour des matériaux différents et incompatibles, pour lesquels il est difficile voire impossible de trouver un matériau commun de soudure ou de collage susceptible d'assurer l'étanchéité parfaite du ballon tout en répondant à d'autres critères .(bio-compatibilité, et...).

Or, il peut arriver que l'on souhaite réaliser l'un des composants du ballon, par exemple l'une des poches ou l'organe d'obturation, avec un nouveau matériau, notamment si ce dernier présente de meilleures propriétés (étanchéité, résistance...) dans l'application considérée.

Dans un tel cas, il est crucial de limiter les répercussions dues à l'usage de ce nouveau matériau sur la fabrication des autres composants du ballon et sur leur assemblage.

Avec les ballons intra-gastriques de l'art antérieur tel, par exemple, celui connu de FR-2834202-A1, de telles répercussions sont difficiles à éviter, dans la mesure où le matériau de soudure ou de collage doit nécessairement être compatible d'une part avec le matériau formant l'organe d'obturation, et d'autre part avec le matériau formant les poches.

### EXPOSE DE L'INVENTION

Les objets assignés à l'invention visent en conséquence à porter remède aux différents inconvénients énumérés précédemment et à proposer un nouveau ballon intra-gastrique expansible pour le traitement de l'obésité dont les composants peuvent être facilement assemblés tout en étant fabriqués à partir de matériaux différents.

Un autre objet de l'invention vise à proposer un nouveau ballon intra-gastrique dont la conception permet de recourir à une large gamme de matériaux pour sa fabrication.

Un autre objet de l'invention vise à proposer un nouveau ballon intra-gastrique dont la fabrication est particulièrement simple et rapide.

Un autre objet de l'invention vise à proposer un nouveau ballon intra-gastrique pourvu d'un organe d'obturation dont l'étanchéité est reproductible avec fiabilité.

Un autre objet de l'invention vise à proposer un nouveau ballon intra-gastrique dont l'étanchéité globale est améliorée.

Un autre objet de l'invention vise à proposer un nouveau ballon intra-gastrique présentant une bonne résistance mécanique générale.

Un autre objet de l'invention vise à proposer un nouveau ballon intra-gastrique dont la structure permet d'améliorer le positionnement du ballon au sein de l'estomac du patient et de limiter les effets indésirables de l'implant sur le fonctionnement de l'appareil digestif.

Les objets assignés à l'invention visent également à proposer un nouveau procédé de fabrication d'un ballon intra-gastrique expansible qui soit reproductible, particulièrement simple et rapide à mettre en oeuvre, tout en permettant d'obtenir un ballon présentant une excellente étanchéité.

Un autre objet de l'invention vise à proposer un nouveau procédé de fabrication d'un ballon intra-gastrique permettant de garantir l'étanchéité de l'organe d'obturation du ballon, et ce de façon systématique et reproductible.

Les objets assignés à l'invention sont atteints à l'aide d'un ballon intra-gastrique expansible, destiné à être implanté au sein de l'estomac d'un patient dans le cadre d'un traitement de l'obésité, et comportant :
- une première poche, suffisamment souple pour passer d'une configuration à volume réduit à une configuration expansée, et pourvue d'au moins un orifice,
- une deuxième poche, disposée de manière à contenir la première poche, et pourvue d'au moins une lumière,
- un organe d'obturation, fixé de façon étanche sur la deuxième poche et destiné à obturer ledit orifice et ladite lumière,
caractérisé en ce que la première et la deuxième poches sont réalisées à partir de matériaux différents non compatibles en matière de soudure ou collage et assemblées à l'aide d'un élément de fixation, adapté pour assurer la fixation étanche de l'organe d'obturation sur la première poche, au sein d'un passage délimité par un col s'étendant à partir de l'orifice, en exerçant une pression suffisante sur ledit col pour le pincer entre l'organe d'obturation et l'élément de fixation.

Les objets assignés à l'invention sont également atteints à l'aide d'un procédé de fabrication d'un ballon intra-gastrique expansible destiné à être implanté au sein de l'estomac d'un patient dans le cadre d'un traitement de l'obésité, dans lequel on prévoit :
- une étape de fabrication d'au moins une première poche, suffisamment souple pour passer d'une configuration à volume réduit à une configuration expansée, et pourvue d'au moins un orifice,
- une étape d'assemblage de la première poche avec une deuxième poche, pourvue d'au moins une lumière, de telle sorte que la première poche soit contenue dans la deuxième poche,
- une étape de fabrication d'un organe d'obturation destiné à obturer ledit orifice et ladite lumière,
- une étape de fixation de l'organe d'obturation sur la deuxième poche de façon sensiblement étanche,
ledit procédé comportant, pour assembler la première et la deuxième poche:
- une étape de montage de l'organe d'obturation au sein d'un passage délimité par un col, s'étendant à partir de l'orifice de la première poche,
- une étape de fixation de l'organe d'obturation sur la première poche de façon étanche à l'aide d'un élément de fixation adapté pour exercer une pression suffisante sur ledit col pour le pincer entre l'organe d'obturation et l'élément de fixation.

### DESCRIPTIF SOMMAIRE DES DESSINS

D'autres objets et avantages de l'invention apparaîtront mieux à la lecture de la description qui suit, ainsi qu'à l'aide des dessins annexés, donnés à titre purement illustratif et non limitatif, dans lesquels :
- La figure 1 illustre, selon une vue transversale en coupe, un premier mode de réalisation d'un ballon intra-gastrique à une poche conforme à l'invention, dans sa configuration expansée.
- Les figures 2 et 3 illustrent, selon une vue en coupe transversale, deux modes de réalisation d'un ballon intra-gastrique à deux poches conforme à l'invention, dans sa configuration expansée.

### MEILLEURE MANIERE DE REALISER L'INVENTION

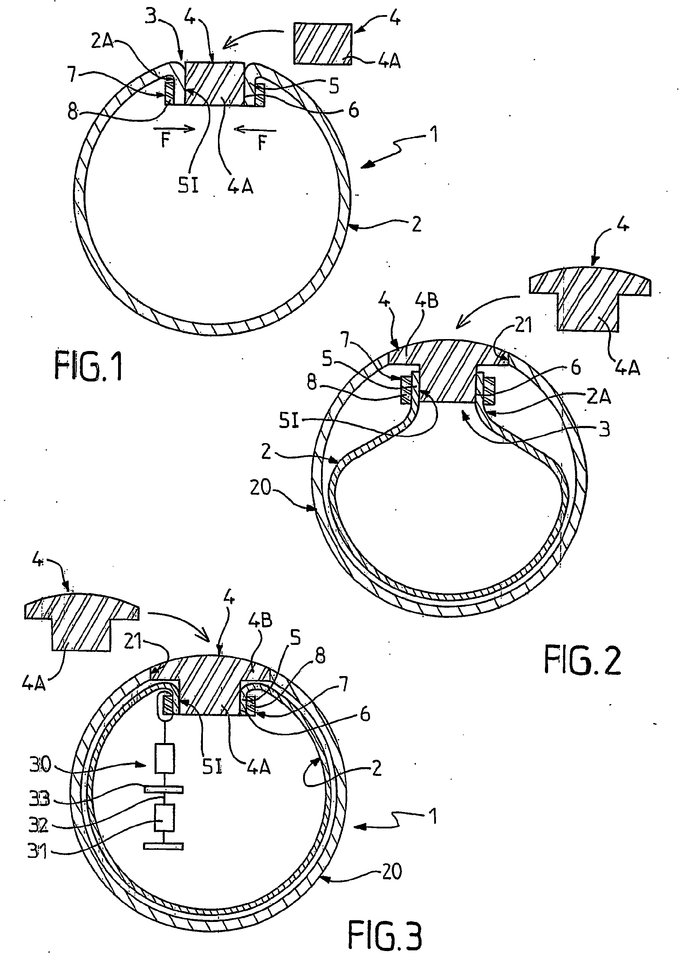

Le ballon intra-gastrique conforme à l'invention va maintenant être décrit en se référant aux figures 1 à 3.

Le ballon 1 intra-gastrique conforme à l'invention est destiné à être implanté dans l'estomac d'un patient dans le cadre d'un traitement de l'obésité.

Le ballon 1 est expansible et comporte à cet effet au moins une première poche 2 suffisamment souple pour passer d'une configuration à volume réduit à une configuration expansée. La configuration à volume réduit peut par exemple correspondre à une configuration dans laquelle le ballon 1 se présente dans une position repliée occupant un volume réduit, facilitant l'introduction du ballon 1 dans l'oesophage.

L'implantation du ballon 1 intra-gastrique conforme à l'invention est en effet généralement réalisée, de manière classique et bien connu de l'homme du métier, par passage dans les voies orales oesophagiennes sous sa forme repliée, comprimée ou lâche. L'expansion, la mise en place et la libération du ballon interviennent à la fin de l'opération gastro-endoscopique, lorsque le ballon intra-gastrique est correctement positionné dans l'estomac du patient.

C'est en effet dans sa configuration expansée que le ballon 1 va pouvoir occuper un volume non négligeable de la cavité gastrique (non représentée), la configuration dite expansée conférant alors au ballon sa forme fonctionnelle, c'est-à-dire la forme d'utilisation thérapeutique du ballon dans le cadre d'un traitement de l'obésité. Ainsi, en occupant une partie du volume interne de l'estomac, le ballon 1 intra-gastrique selon l'invention permet de créer chez le patient une sensation rapide de satiété, qui s'accompagne généralement d'une diminution de la quantité d'aliments ingérés.

Selon l'invention, la première poche 2 comporte au moins un orifice 3, qui peut résulter du procédé de fabrication de la première poche 2, ou encore être spécifiquement ménagé dans ladite première poche 2 en vue par exemple d'introduire un fluide de gonflage au sein de cette dernière.

Selon l'invention, le ballon 1 comporte également un organe d'obturation 4 de ladite première poche 2, destiné à venir obturer l'orifice 3. La première poche 2 est en effet préférentiellement destinée à être remplie à l'aide d'un fluide de gonflage, par exemple un gaz ou un liquide, et l'organe d'obturation 4 est conçu pour assurer l'étanchéité de la première poche 2 une fois cette dernière gonflée.

Selon l'invention, la première poche 2 est pourvue d'un col 5, lequel s'étend à partir de l'orifice 3 pour délimiter un passage 6 entre l'intérieur et l'extérieur de la première poche 2. Le passage 6 est ainsi avantageusement adapté pour recevoir l'organe d'obturation 4.

Le col 5 est avantageusement formé par un repli 2A de la paroi de la première poche 2, agencé de manière à s'étendre sensiblement tout autour de l'organe d'obturation 4.

Selon l'invention, le ballon 1 comporte un élément de fixation 7 destiné à assurer la fixation de l'organe d'obturation 4 sur la première poche 2. A cet effet, l'élément de fixation 7 est adapté pour venir exercer une pression sur le col 5 tendant à refermer ce dernier sur l'organe d'obturation 4.

L'élément de fixation 7 est en particulier adapté pour exercer une pression suffisante sur le col 5, pour pincer ce dernier entre l'organe d'obturation 4 et l'élément de fixation 7 assurant ainsi d'une part la fixation de l'organe d'obturation 4 sur la première poche 2, et d'autre part l'étanchéité de cette dernière au niveau de l'orifice 3.

Le ballon 1 intra-gastrique ainsi conçu permet alors un montage simple et fiable de l'organe d'obturation 4 sur la première poche 2 alors même que ces deux éléments sont fabriqués à partir de matériaux différents dont l'assemblage classique par collage ou à l'aide de soudures s'avèrerait particulièrement difficile. -

Un tel montage permet ainsi de réaliser un assemblage résistant des composants du ballon et ce, sans avoir nécessairement recours à de multiples soudures ou collages.

Avantageusement, le col 5 comporte une paroi interne 5l qui délimite le passage 6. L'élément de fixation 7 est ainsi avantageusement disposé de manière à ceinturer le col 5 et à venir exercer une pression sensiblement homogène sur la circonférence de ce dernier telle que la paroi interne 5l du col 5 vienne sensiblement épouser, avec étanchéité, l'organe d'obturation 4.

De façon particulièrement avantageuse, la pression exercée par l'élément de fixation 7 est orientée suivant une direction centripète (flèches F sur la figure 1).

Avantageusement, l'organe d'obturation 4 comprend un septum 4A à effet auto-obturant, disposé au sein du passage 6 sensiblement en regard de l'élément de fixation 7. Le col 5 et le septum 4A sont ainsi avantageusement conformés pour que le col 5 vienne sensiblement entourer le septum 4A dans sa position d'obturation de la première poche 2.

De façon particulièrement avantageuse, l'élément de fixation 7 est conçu pour comprimer suffisamment le septum 4A pour assurer l'étanchéité de ce dernier vis-à-vis des fluides susceptibles d'être contenus dans la première poche 2. Ainsi, en exerçant une pression sur le col 5, l'élément de fixation 7 vient en même temps comprimer le septum 4A pour assurer son étanchéité.

L'élément de fixation 7 assure ainsi avantageusement et simultanément une double fonction :
- d'une part il assure l'assemblage de l'organe d'obturation 4 sur la première poche 2,
- et d'autre part il confère à l'organe d'obturation 4 sa propre fonction d'étanchéité, en assurant la compression du septum 4A.

Pour cette raison, la pression exercée par l'élément de fixation 7 sur le col 5 devra être suffisante pour :
- d'une part assurer l'étanchéité entre le col 5 et l'organe d'obturation 4,
- et d'autre part assurer l'étanchéité du septum 4A.

Le montage précédemment décrit permet donc de limiter le nombre de pièces nécessaires pour assurer les mêmes fonctions.

De façon particulièrement avantageuse, l'élément de fixation 7 est formé par une bague 8 se présentant de préférence sous la forme d'un tube cylindrique et disposée de manière à entourer le col 5. La bague 8 est de préférence sensiblement rigide et dimensionnée pour comprimer suffisamment le septum 4A en direction centripète afin d'obtenir une étanchéité suffisante de ce dernier. Le diamètre de la bague 8 devra en particulier être calculé en fonction du taux de compression du septum 4A nécessaire pour obtenir une bonne étanchéité.

De façon particulièrement avantageuse, la première poche 2 et l'organe d'obturation 4 sont réalisés à partir de matériaux différents, et préférentiellement à partir d'élastomères.

Plusieurs modes de réalisation de l'invention vont maintenant être décrits en s'appuyant sur les figures 1 à 3.

Selon un premier mode de réalisation de l'invention représenté sur la figure 1, le ballon 1 intra-gastrique ne comporte qu'une seule poche 2 avec un orifice 3 obturé par un organe d'obturation 4. Dans ce mode de réalisation de l'invention, l'organe d'obturation 4 est préférentiellement formé par le septum 4A, lequel vient se loger à l'intérieur du col 5 qui s'étend, à partir de l'orifice 3, vers l'intérieur de la poche 2, formant ainsi un col rentrant.

La poche 2 est préférentiellement réalisée à partir de polyuréthane élastomère, et présente donc de bonnes propriétés de souplesse et d'élasticité. Le septum 4A est quant à lui préférentiellement réalisé à base de silicone. L'élément de fixation 7, précisément la bague 8 vient alors entourer le col 5 et comprimer le septum 4A assurant ainsi d'une part l'étanchéité de ce dernier, et d'autre part sa fixation étanche sur la poche 2, par pincement du col 5 entre le septum 4A et la bague 8 (figure 1).

Avantageusement, le septum 4A ne fait pas saillie à l'extérieur de la poche 2, conférant ainsi au ballon 1 intra-gastrique un caractère atraumatique.

Selon un deuxième et un troisième modes de réalisation de l'invention représentés sur les figures 2 et 3, le ballon 1 intra-gastrique comporte avantageusement une deuxième poche 20 souple, disposée de manière à contenir la première poche 2, formant ainsi l'enveloppe externe d u ballon.

De façon particulièrement avantageuse, et tel que cela est représenté sur les figures 2 et 3, l'élément de fixation 7 est adapté pour assurer l'assemblage de la première poche 2, interne, avec la deuxième poche 20, externe.

A cet effet, la deuxième poche 20 est avantageusement pourvue d'au moins une lumière 21, et l'organe d'obturation 4 est adapté pour obturer de façon sensiblement étanche ladite lumière 21. Ainsi, l'organe d'obturation 4 comporte de préférence une collerette 4B destinée à permettre la fixation étanche de l'organe d'obturation 4 sur la deuxième poche 20, par exemple par collage ou soudure de la collerette 48 sur le pourtour de la lumière 21.

De façon préférentielle, le matériau formant l'organe d'obturation 4 est compatible avec le matériau constituant la deuxième poche 20, au moins au niveau du pourtour de la lumière 21, de manière à permettre la fixation dudit organe de d'obturation 4 sur la deuxième poche 20 par soudure ou collage.

L'organe d'obturation 4 est ainsi avantageusement fixé directement sur la deuxième poche 20 par soudure ou collage.

De façon préférentielle, l'organe d'obturation 4 est formé par un matériau sensiblement de même nature chimique et physique que le matériau formant la deuxième poche 20.

De façon encore plus préférentielle, l'organe d'obturation 4 et la deuxième poche 20 sont réalisés à partir du même matériau, tel que du silicone.

Selon un mode de réalisation possible de l'invention, non représenté aux figures, l'organe d'obturation 4 et la deuxième poche 20 sont venus de matière et forment un ensemble monobloc, susceptible d'être obtenu par moulage.

Avantageusement, la collerette 4B est disposée de manière à recouvrir le col 5 de la première poche 2 et l'élément de fixation 7, afin de protéger l'assemblage de la première poche 2 avec l'organe d'obturation 4 et d'éviter leur désolidarisation, par exemple sous l'effet du mouvement du ballon intra-gastrique 1 dans l'estomac. En effet, si le col 5 et/ou l'élément de fixation 7 faisaient saillie à l'extérieur du ballon, les mouvements répétés du ballon à l'intérieur de l'estomac pourraient entraîner le desserrement progressif de l'élément de fixation 7, ce que la collerette 4B permet d'éviter.

L'élément de fixation 7 assure ainsi une troisième fonction : assembler la première et la deuxième poches 2, 20 et ce, de façon particulièrement fiable et reproductible quelle que soit la nature des matériaux formant lesdites poches 2, 20. Il est ainsi possible, avec le présent montage, d'utiliser deux matériaux différents et non nécessairement compatibles en matière de soudure ou collage pour réaliser la poche interne et l'enveloppe externe et ce, sans contrainte de fabrication supplémentaire.

De façon préférentielle, la première et la deuxième poches 2, 20 sont réalisées à partir de matériaux différents non compatibles, la première poche 2 étant de préférence en polyuréthane, alors que la deuxième poche 20 est préférentiellement en silicone.

Avantageusement, la première poche 2, interne, est réalisée à partir d'un matériau présentant de meilleures propriétés d'étanchéité que le matériau formant la deuxième poche 20, externe.

Grâce aux bonnes propriétés d'étanchéité du polyuréthane vis-à-vis des gaz, il est possible de réduire l'épaisseur de la première poche 2, ce qui a pour effet direct de diminuer sensiblement l'encombrement du ballon 1 dans sa configuration à volume réduit, facilitant ainsi son implantation.

Dans les deux modes de réalisation représentés sur les figures 2 et 3, l'organe d'obturation 4 est avantageusement formé par une valve en silicone dont une partie, destinée à venir se loger au sein du col 5, est constituée par le septum 4A en silicone.

Un tel organe d'obturation 4 autorise ainsi la traversée du septum 4A par une aiguille de gonflage, permettant ainsi le remplissage de la première poche 2, le caractère auto-obturant de l'organe d'obturation 4, précisément du septum 4A, assurant l'étanchéité du système lorsque l'aiguille de gonflage est retirée.

Selon le troisième mode de réalisation représenté sur la figure 3, le col 5 s'étend, à partir de l'orifice 3, vers l'intérieu r de la première poche 2, formant ainsi un col rentrant. Grâce à cette configuration, la deuxième poche 20 vient avantageusement épouser la forme de la première poche 2 lorsque cette dernière est remplie avec le fluide de gonflage. Le ballon 1 présente alors une structure sensiblement compacte et donc plus résistante mécaniquement.

Bien évidemment, il est également possible de réaliser un ballon 1 intra-gastrique dans lequel le col 5 de la première poche 2 s'étend, à partir de l'orifice 3, vers l'extérieur de ladite première poche 2 de manière à former un col sortant (deuxième mode de réalisation représenté sur la figure 2), et ce, sans sortir du cadre de l'invention.

De façon particulièrement avantageuse, le ballon 1 intra-gastrique comporte un moyen de lestage 30 destiné à alourdir sensiblement le ballon 1 (figure 3).

Ainsi, lorsque la première poche 2 est uniquement remplie de gaz, le ballon 1 peut avoir tendance, en raison de son faible poids, à remonter dans la partie haute de l'estomac, gênant ainsi la pénétration des aliments dans la cavité gastrique.

Au contraire, si la première poche 2 est uniquement remplie dé liquide, le ballon 1 intra-gastrique risque d'être trop lourd, et donc mal supporté par le patient. L'utilisation du moyen de lestage 30 constitue ainsi un compromis permettant d'améliorer le positionnement du ballon 1 au sein de l'estomac et d'éliminer en même temps une source d'inconfort pour le patient.

Avantageusement, le moyen de lestage 30 est formé par une pluralité de corps solides et denses 31 reliés entre eux par des portions de fil 32.

De façon préférentielle, le moyen de lestage 30 comporte également des entretoises 33 disposées entre deux corps solides et denses 31 consécutifs de manière à éviter les chocs, et donc les bruits indésirables. Les entretoises 33 sont de préférence en élastomère, par exemple en silicone.

Les corps solides et denses 31 sont préférentiellement fabriqués à base de tungstène, préféré notamment en raison de son caractère bio-compatible.

De façon particulièrement avantageuse, et tel que cela est représenté sur la figure 3, le moyen de lestage 30 est de préférence disposé à l'intérieur de la première poche 2 et l'une des extrémités du fil reliant les corps solides et denses 31 est solidarisée avec l'élément de fixation 7, ce qui permet de limiter la mobilité du moyen de lestage 30 au sein du ballon 1.

L'élément de fixation 7 peut ainsi assurer avantageusement une quatrième fonction, à savoir celle de support et de moyen d'attache pour le moyen de lestage 30.

L'invention concerne également un procédé de fabrication d'un ballon 1 intra-gastrique expansible destiné à être implanté au sein de l'estomac d'un patient dans le cadre d'un traitement de l'obésité, dans lequel on prévoit les étapes :
- de fabrication d'au moins une première poche 2, suffisamment souple pour passer d'une configuration à volume réduit à une configuration expansée, et pourvue d'au moins un orifice 3,
- et de fabrication d'un organe d'obturation 4 de ladite première poche 2, destiné à venir obturer ledit orifice 3.

Avantageusement, l'étape de fabrication de la première poche 2 comprend une sous-étape dans laquelle on fabrique, par exemple par thermoformage, deux hémisphères dont on vient ensuite souder (ou coller) les frontières de manière à former une première poche 2 sensiblement sphérique et munie d'un col 5.

Plus précisément, le procédé de fabrication de la première poche 2 comporte une étape au cours de laquelle on assemble par soudure ou collage le long d'une ligne de soudure périphérique, une ou plusieurs feuilles (par exemple deux feuilles) de forme prédéterminée en matériau sensiblement imperméable aux gaz, tel que le polyuréthane thermoplastique élastomère, préalablement mises en forme, par exemple par thermoformage, de manière à leur conférer une forme hémisphérique.

Chaque feuille peut être formée par un unique film de polyuréthane, mais est de préférence formée par plusieurs, et par exemple deux films de polyuréthane superposés, lesdits films pouvant être solidarisés ensemble, par exemple par collage, ou mobiles les uns par rapport aux autres.

De façon préférentielle, chaque feuille comporte un prolongement, par exemple de forme rectangulaire. Lors de l'assemblage des feuilles, les prolongements se superposent et sont soudés ou collés ensemble, le long d'une ligne de soudure (ou collage) périphérique, de manière à former le col 5 de la première poche 2.

Bien évidemment, l'étape de fabrication de la première poche 2 peut également résulter d'un autre procédé, tel qu'un procédé de thermoscellage, et ce sans sortir du cadre de l'invention.

Selon l'invention, le procédé comporte ensuite une étape (a) de pincement du col 5 entre l'organe d'obturation 4 et un élément de fixation 7 adapté, de manière à assurer d'une part la fixation de l'organe d'obturation 4 sur la première poche 2, et d'autre part l'étanchéité de cette dernière.

Ce procédé permet ainsi de supprimer avantageusement l'étape classique de soudure ou de collage de l'organe d'obturation 4 sur la première poche 2, accélérant ainsi l'opération de fabrication du ballon 1. En outre, ce procédé est particulièrement avantageux dans les cas où la première poche 2 et l'organe d'obturation 4 sont réalisés à partir de matériaux non compatibles, c'est-à-dire des matériaux dont l'assemblage par soudure ou par collage est difficile voire impossible avec une garantie de qu alité.

De façon particulièrement avantageuse, le procédé comporte une étape (b) de retournement du col 5 de telle sorte que ce dernier se situe à l'intérieur de la première poche 2 et forme un col rentrant. L'étape (b) de retournement sera préférentiellement effectuée avant la soudure complète des deux hémisphères sus-mentionnés de manière à con server un accès à l'intérieur de la première poche 2.

Avantageusement, le procédé comprend ensuite une étape (c) de montage de l'organe d'obturation 4 au sein du passage 6 formé par le col 5, suivie d'une étape (d) de montage d'une bague 8 autour du col 5, ladite bague 5 formant l'élément de fixation 7, de manière à ceinturer le col 5 et à exercer une pression sur la circonférence de ce dernier telle que la paroi interne 5l du col 5 vienne sensiblement épouser, avec étanchéité, l'organe d'obturation 4.

Avantageusement, le procédé comporte également une étape (e) d'assemblage de la première poche 2 avec une deuxième poche 20, dans laquelle on fixe l'organe d'obturation 4 sur la deuxième poche 20 de façon sensiblement étanche. A cet effet, l'étape (e) d'assemblage comporte une sous-étape dans laquelle on vient souder ou coller la collerette 4B de l'organe d'obturation 4 avec le pourtour de la lumière 21 ménagée dans la deuxième poche 20.

Une telle conception à deux poches 2, 20 permet ainsi de constituer un ballon 1 intra-gastrique qui, tout en conservant une structure souple et élastique, présente une résistance mécanique améliorée. Ainsi, la première poche 2 constitue alors de préférence une chambre de gonflage et est à ce titre destinée à être remplie avec un fluide de gonflage, par exemple de l'air, la deuxième poche 20 constituant alors l'enveloppe externe protectrice du ballon 1, dont la mise en forme, et en particulier le déploiement est commandé par la première poche 2. Ainsi, au fur et à mesure du gonflage de la première poche 2, cette dernière va venir repousser la paroi de la deuxième poche 20, et ce jusqu'à ce que ladite deuxième poche 20 atteigne sa forme fonctionnelle au sein de l'estomac.

Grâce à sa conception particulière, le ballon 1 intra-gastrique conforme à l'invention est susceptible de suivre aisément les évolutions techniques dans le domaine, et notamment les développements concernant les matériaux utilisés dans la fabrication des ballons.

L'invention permet donc de réaliser un ballon dont les composants, notamment les poches et l'organe d'obturation, peuvent être facilement assemblés tout en étant indépendants les uns des autres sur le plan structurel et fabriqués à partir de matériaux distincts non nécessairement compatibles.

Un autre avantage du ballon 1 intra-gastrique conforme à l'invention est qu'il bénéficie d'une bonne reproductibilité, d'une part dans l'assemblage de ses différents composants, et d'autre part dans ses fonctionnalités. En particulier, l'élément de fixation 7 est dimensionné pour garantir de façon reproductible le caractère obturant de l'organe d'obturation 4, et ce en calculant au préalable le taux de compression que l'élément de fixation 7 doit exercer sur le septum 4A pour assurer l'étanchéité de ce dernier.

Un autre avantage du ballon 1 intra-gastrique conforme à l'invention provient du fait qu'une même pièce, à savoir l'élément de fixation 7, peut être à même d'assurer plusieurs fonctions distinctes et notamment :
1) l'assemblage de l'organe d'obturation 4 sur la première poche 2,
2) l'étanchéité de l'organe d'obturation 4, précisément du septum 4A,
3) l'assemblage de la première poche 2 avec la deuxième poche 20,
4) la fixation du moyen de lestage 30.

### POSSIBILITE D'APPLICATION INDUSTRIELLE

L'invention trouve son application industrielle dans la conception et la fabrication de dispositifs implantables de lutte contre l'obésité.

## Revendications

1. Ballon intra-gastrique expansible, destiné à être implanté au sein de l'estomac d'un patient dans le cadre d'un traitement de l'obésité, et comportant :
- une première poche (2), suffisamment souple pour passer d'une configuration à volume réduit à une configuration expansée, et pourvue d'au moins un orifice (3),
- une deuxième poche (20), disposée de manière à contenir la première poche (2), et pourvue d'au moins une lumière (21),
- un organe d'obturation (4), fixé de façon étanche sur la deuxième poche (20) et destiné à obturer ledit orifice (3) et ladite lumière (21),
**caractérisé en ce que** la première et la deuxième poches (2, 20) sont réalisées à partir de matériaux différents non compatibles en matière de soudure ou collage et assemblées à l'aide d'un élément de fixation (7), adapté pour assurer la fixation étanche de l'organe d'obturation (4) sur la première poche (2), au sein d'un passage (6) délimité par un col (5) s'étendant à partir de l'orifice (3), en exerçant une pression suffisante sur ledit col (5) pour le pincer entre l'organe d'obturation (4) et l'élément de fixation (7).

2. Ballon intra-gastrique selon la revendication 1 **caractérisé en ce que** l'organe d'obturation (4) et la deuxième poche (20) sont réalisés à partir de matériaux compatibles, et **en ce que** l'organe d'obturation (4) est fixé sur la deuxième poche (20) par soudure ou collage.

3. Ballon intra-gastrique selon la revendication 1 ou 2 **caractérisé en ce que** l'organe d'obturation (4) comporte une collerette (4B) permettant sa fixation étanche sur le pourtour de la lumière (21), par exemple par collage ou soudure de la collerette (4B) avec le pourtour de la lumière (21).

4. Ballon intra-gastrique selon la revendication 1, 2 ou 3 **caractérisé en ce que** l'organe d'obturation (4) comprend un septum (4A), disposé au sein du passage (6) sensiblement en regard de l'élément de fixation (7).

5. Ballon intra-gastrique selon la revendication 4 **caractérisé en ce que** l'élément de fixation (7) est adapté pour comprimer suffisamment le septum (4A) pour assurer l'étanchéité de ce dernier vis-à-vis des fluides susceptibles d'être contenus dans la première poche (2).

6. Ballon intra-gastrique selon l'une des revendications précédentes **caractérisé en ce que** le col (5) s'étend, à partir de l'orifice (3), vers l'intérieur de la première poche (2).

7. Ballon intra-gastrique selon l'une des revendications précédentes **caractérisé en ce que** le col (5) comportant une paroi interne (51) délimitant ledit passage (6), l'élément de fixation (7) est disposé de manière à ceinturer le col (5) et à exercer une pression sur la circonférence de ce dernier telle que la paroi interne (51) du col (5) vienne sensiblement épouser, avec étanchéité, l'organe d'obturation (4).

8. Ballon intra-gastrique selon l'une des revendications 1 à 7 **caractérisé en ce que** l'élément de fixation (7) est formé par une bague (8).

9. Ballon intra-gastrique selon l'une des revendications 1 à 8 **caractérisé en ce que** la première poche (2) est en polyuréthane, la deuxième poche (20) étant en silicone.

10. Ballon intra-gastrique selon l'une des revendications précédentes **caractérisé en ce que** l'organe d'obturation (4) est formé par une valve en silicone.

11. Ballon intra-gastrique selon l'une des revendications 1 à 10 **caractérisé en ce qu'**il comporte un moyen de lestage (30) destiné à alourdir sensiblement le ballon.

12. Ballon intra-gastrique selon la revendication 11 **caractérisé en ce que** le moyen de lestage (30) est formé par une pluralité de corps solides et denses (31) reliés entre eux par des portions de fil (32).

13. Ballon intra-gastrique selon la revendication 12 **caractérisé en ce que** le moyen de lestage (30) comporte des entretoises (33), disposées entre deux corps (31) consécutifs de manière à éviter les chocs.

14. Ballon intra-gastrique selon l'une des revendications 11 à 13 **caractérisé en ce que** le moyen de lestage (30) est supporté par l'élément de fixation (7).

15. Ballon intra-gastrique selon les revendications 12 et 14 **caractérisé en ce que** l'une des extrémités du fil reliant les corps (31) est solidarisée avec l'élément de fixation (7).

16. Ballon intra-gastrique selon l'une des revendications 11 à 15 **caractérisé en ce que** le moyen de lestage (30) est disposé à l'intérieur de la première poche (2).

17. Procédé de fabrication d'un ballon (1) intra-gastrique expansible selon l'une des revendications 1 à 16, dans lequel on prévoit :
- une étape de fabrication d'au moins une première poche (2), suffisamment souple pour passer d'une configuration à volume réduit à une configuration expansée, et pourvue d'au moins un orifice (3),
- une étape d'assemblage de la première poche (2) avec une deuxième poche (20), pourvue d'au moins une lumière (21), de telle sorte que la première poche (2) soit contenue dans la deuxième poche (20),
- une étape de fabrication d'un organe d'obturation (4) destiné à obturer ledit orifice (3) et ladite lumière (21),
- une étape de fixation de l'organe d'obturation (4) sur la deuxième poche (20) de façon sensiblement étanche,
ledit procédé comportant, pour assembler la première et la deuxième poche (2, 20):
- une étape de montage de l'organe d'obturation (4) au sein d'un passage (6) délimité par un col (5), s'étendant à partir de l'orifice (3) de la première poche (2),
- une étape de fixation de l'organe d'obturation (4) sur la première poche (2) de façon étanche à l'aide d'un élément de fixation (7) adapté pour exercer une pression suffisante sur ledit col (5) pour le pincer entre l'organe d'obturation (4) et l'élément de fixation (7).

18. Procédé selon la revendication 17 **caractérisé en ce que** l'étape de fixation de l'organe d'obturation (4) sur la deuxième poche (20) s'effectue par soudure ou collage.

19. Procédé selon la revendication 17 ou 18 **caractérisé en ce que** l'organe d'obturation (4) comporte une collerette (4B) et **en ce que** le procédé comporte une sous-étape dans laquelle on vient souder ou coller ladite collerette (4B) avec le pourtour de la lumière (21).

20. Procédé selon l'une des revendications 17 à 19 **caractérisé en ce qu'**il comporte une étape (b) de retournement du col (5) de telle sorte que ce dernier se situe à l'intérieur de la première poche (2).

## Claims

1. Expandable intragastric balloon designed to be implanted inside the stomach of a patient for the treatment of obesity, and comprising:
- a first pouch (2) that is sufficiently flexible to pass from a reduced-volume configuration to an expanded configuration, and provided with at least one port (3),
- a second pouch (20) arranged so as to contain the first pouch (2), and provided with at least one hole (21),
- a sealing member (4) fastened onto the second pouch (20) in a leak-proof manner, and designed to seal said port (3) and said hole (21),
**characterized in that** the first and the second pouches (2, 20) are made of different, non-compatible materials in terms of welding or gluing, and are assembled together with the aid of a fastening element (7) designed to ensure the leak-proof fastening of the sealing member (4) onto the first pouch (2), inside a passage (6) defined by a neck (5) extending from the port (3), by exerting a sufficient amount of pressure on said neck (5) in order to pinch it between the sealing member (4) and the fastening element (7).

2. Intragastric balloon of claim 1, **characterized in that** the sealing member (4) and the second pouch (20) are made of compatible materials, and **in that** the sealing member (4) is fastened onto the second pouch (20) by welding or gluing.

3. Intragastric balloon as claimed in claim 1 or 2, **characterized in that** the sealing member 4 comprises a flange (4B) enabling it to be fastened onto the periphery of the hole (21) in a leak-proof manner, e.g., by gluing or welding the flange (4B) together with the periphery of the hole (21).

4. Intragastric balloon as claimed in claim 1, 2 or 3, **characterized in that** the sealing member (4) comprises a septum (4A) arranged inside the passage (6) substantially opposite the fastening element (7).

5. Intragastric balloon as claimed in claim 4, **characterized in that** the fastening element (7) is designed to sufficiently compress the septum (4A) in order to ensure the leak tightness thereof, with respect to the fluids likely to be contained inside the first pouch (2).

6. Intragastric balloon as claimed in one of the preceding claims, **characterized in that** the neck (5) extends from the port (3) towards the interior of the first pouch (2).

7. Intragastric balloon as claimed in one of the preceding claims, **characterized in that** the neck (5) comprising an internal wall (51) defining said passage (6), the fastening element (7) is arranged so as to surround the neck (5) and to exert pressure on the circumference thereof, such that the internal wall (51) of the neck (5) conforms in shape substantially to the sealing member (4), in a leak-proof manner.

8. Intragastric- balloon as claimed in one of claims 1 to 7, **characterized in that** the fastening element (7) consists of a ring (8).

9. Intragastric balloon as claimed in one of claims 1 to 8, **characterized in that** the first pouch (2) is made of polyurethane, the second pouch (20) being made of silicone.

10. Intragastric balloon as claimed in one of the preceding claims, **characterized in that** the sealing member (4) consists of a silicone valve.

11. InLragastric balloon as claimed in one of claims 1 to 10, **characterized in that** it comprises a ballasting means (30) designed to substantially weigh down the balloon.

12. Intragastric balloon of claim 11, **characterized in that** the ballasting means (30) consist of a plurality of solid and dense bodies (31) joined together by thread portions (32).

13. Intragastric balloon of claim 12, **characterized in that** the ballasting means (30) comprise spacers (33) arranged between two consecutive bodies (31) so as to prevent shocks.

14. Intragastric balloon as claimed in one of claims 11 to 13, **characterized in that** the ballasting means (30) is supported by the fastening element (7).

15. Intragastric balloon as claimed in claims 12 and 14, **characterized in that** one of the ends of the thread joining together the bodies (31) is firmly attached to the fastening element (7).

16. Intragastric balloon as claimed in one of claims 11 to 15, **characterized in that** the ballasting means (30) is arranged inside the first pouch (2).

17. Method of manufacturing an expandable intragastric balloon (1) as claimed in one of claims 1 to 16, wherein provisions are made for:
- a step for manufacturing at least one first pouch (2) that is sufficiently flexible to pass from a reduced-volume configuration to an expanded configuration, and that is provided with at least one port (3),
- a step for assembling the first pouch (2) together with a second pouch (20), provided with at least one hole (21), with the result being that the first pouch (2) is contained inside the second pouch (20),
- a step for manufacturing a sealing member (4) designed to seal said port (3) and said hole (21),
- a step for fastening the sealing member (4) onto the second pouch (20) in a substantially leak-proof manner,
said method comprising, in order to assemble the first and the second pouch (2, 20):
- a step for mounting the sealing member (4) inside a passage (6) defined by a neck (5), extending from the port (3) of the first pouch (2),
- a step for fastening the sealing member (4) onto the first pouch (2) in a leak-proof manner with the aid of a fastening element (7) suitable for exerting sufficient pressure on the neck (5) in order to pinch it between the sealing member (4) and the fastening element (7).

18. Method of claim 17, **characterized in that** the step for fastening the sealing member (4) onto the second pouch (20) is carried out by welding or gluing.

19. Method as claimed in claim 17 or 18, **characterized in that** the sealing member (4) comprises a flange (4B) and **in that** the method comprises a sub-step wherein said flange (4B) is welded or glued together with the periphery of the hole (21).

20. Method as claimed in one of claims 17 to 19, **characterized in that** it comprises a step (b) for turning over the neck (5) such that it is situated inside the first pouch (2).

## Patentansprüche

1. Dehnbarer Magenballon, dazu bestimmt, innerhalb des Magens eines Patienten im Rahmen einer Behandlung von Übergewichtigkeit implantiert zu sein, und umfassend:
- einen ersten Beutel (2), welcher ausreichend flexibel ist, von einer Form mit reduziertem Volumen in eine gedehnte Form überzugehen, und welcher mit wenigstens einer Öffnung (3) versehen ist,
- einen zweiten Beutel (20), welcher derart angeordnet ist, dass er den ersten Beutel (2) enthält, und welcher mit wenigstens einem Loch (21) versehen ist,
- ein Verschlussorgan (4), welches auf dichte Weise an dem zweiten Beutel (20) befestigt ist, und welches dazu bestimmt ist, die Öffnung (3) und das Loch (21) zu verschließen,
**dadurch gekennzeichnet, dass** der erste und der zweite Beutel (2, 20) aus unterschiedlichen Materialien, welche, in Bezug auf Verschweißen und Verkleben, nicht kompatibel sind, hergestellt sind, und dass sie mit Hilfe eines Befestigungselements (7) zusammengebaut sind, welches angepasst ist, die dichte Befestigung des Verschlussorgans (4) an dem ersten Beutel (2) innerhalb eines Durchgangs (6), der durch einen Kragen (5) begrenzt ist, der sich von der Öffnung (3) aus erstreckt, sicherzustellen, indem es einen ausreichenden Druck auf den Kragen (5) ausübt, um ihn zwischen dem Verschlussorgan (4) und dem Befestigungselement (7) einzuklemmen.

2. Magenballon nach Anspruch 1, **dadurch gekennzeichnet, dass** das Verschlussorgan (4) und der zweite Beutel (20) aus kompatiblen Materialien hergestellt sind, und dadurch, dass das Verschlussorgan (4) an dem zweiten Beutel (20) durch Verschweißen oder Verkleben befestigt ist.

3. Magenballon nach Anspruch 1 oder 2, **dadurch gekennzeichnet, dass** das Verschlussorgan (4) einen Rand (4B) aufweist, welcher dichte Befestigung desselben an der Umrandung des Lochs (21) erlaubt, zum Beispiel durch Verkleben oder Verschweißen des Rands (4B) mit der Umrandung des Lochs (21) .

4. Magenballon nach Anspruch 1, 2 oder 3, **dadurch gekennzeichnet, dass** das Verschlussorgan (4) ein Septum (4A) umfasst, welches innerhalb eines Durchgangs (6) im Wesentlichen gegenüber dem Befestigungselement (7) angeordnet ist.

5. Magenballon nach Anspruch 4, **dadurch gekennzeichnet, dass** das Befestigungselement (7) angepasst ist, das Septum (4A) ausreichend zusammenzudrücken, um die Dichtheit des Letzteren gegen Fluide, die geeignet sind, in dem ersten Beutel (2) enthalten zu sein, sicherzustellen.

6. Magenballon nach einem der vorhergehenden Ansprüche, **dadurch gekennzeichnet, dass** der Kragen (5) sich von der Öffnung (3) aus in das Innere des ersten Beutels (2) erstreckt.

7. Magenballon nach einem der vorhergehenden Ansprüche, **dadurch gekennzeichnet, dass** der Kragen (5) eine Innenwand (51) aufweist, welche den Durchgang (6) begrenzt, das Befestigungselement (7) derart angeordnet ist, dass es den Kragen (5) umschließt und einen Druck auf den Umfang des Letzteren ausübt, derart, dass die Innenwand (51) des Kragens (5) an dem Verschlussorgan (4) im wesentlichen dicht anliegen wird.

8. Magenballon nach einem der Ansprüche 1 bis 7, **dadurch gekennzeichnet, dass** das Befestigungselement (7) durch einen Ring (8) gebildet ist.

9. Magenballon nach einem der Ansprüche 1 bis 8, **dadurch gekennzeichnet, dass** der erste Beutel (2) aus Polyurethan besteht, wobei der zweite Beutel (20) aus Silikon besteht.

10. Magenballon nach einem der vorhergehenden Ansprüche, **dadurch gekennzeichnet, dass** das Verschlussorgan (4) durch ein Ventil aus Silikon gebildet ist.

11. Magenballon nach einem der Ansprüche 1 bis 10, **dadurch gekennzeichnet, dass** er ein Ballastmittel (30) aufweist, welches dazu bestimmt ist, den Ballon im Wesentlichen zu beschweren.

12. Magenballon nach Anspruch 11, **dadurch gekennzeichnet, dass** das Ballastmittel (30) durch mehrere feste und dichte Körper (31) gebildet ist, welche miteinander durch Fadenabschnitte (32) verbunden sind.

13. Magenballon nach Anspruch 12, **dadurch gekennzeichnet, dass** das Ballastmittel (30) Zwischenstücke (33) umfasst, welche zwischen zwei aufeinanderfolgenden Körpern (31) angeordnet sind, derart, dass Zusammenstöße vermieden werden.

14. Magenballon nach einem der Ansprüche 11 bis 13, **dadurch gekennzeichnet, dass** das Ballastmittel (30) durch das Befestigungselement (7) getragen ist.

15. Magenballon nach Anspruch 12 und 14, **dadurch gekennzeichnet, dass** eines der Enden des Fadens, welcher die Körper (31) verbindet, mit dem Befestigungselement (7) verbunden ist.

16. Magenballon nach einem der Ansprüche 11 bis 15, **dadurch gekennzeichnet, dass** das Ballastmittel (30) im Inneren des ersten Beutels (2) angeordnet ist.

17. Verfahren zur Herstellung eines dehnbaren Magenballons (1) nach einem der Ansprüche 1 bis 16, wobei vorgesehen sind:
- ein Schritt des Herstellens wenigstens eines ersten Beutels (2), welcher ausreichend flexibel ist, von einer Form mit reduziertem Volumen in eine gedehnte Form überzugehen, und welcher mit wenigstens einer Öffnung (3) versehen ist,
- ein Schritt des Zusammenbauens des ersten Beutels (2) mit einem zweiten Beutel (20), welcher mit wenigstens einem Loch (21) versehen ist, derart, dass der erste Beutel (2) in dem zweiten Beutel (20) enthalten ist,
- ein Schritt des Herstellens eines verschlussorgans (4), welches dazu bestimmt ist, die Öffnung (3) und das Loch (21) zu verschließen,
- ein Schritt des Befestigen des Versahlussorgans (4) an dem zweiten Beutel (20) auf eine im Wesentlichen dichte Weise,
wobei das Verfahren, zum Zusammenbauen des ersten und des zweiten Beutels (2, 20), umfasst:
- einen Schritt des Anbringens des Verschlussorgans (4) innerhalb eines Durchgangs (6), welcher durch einen Kragen (5) begrenzt ist, der sich von der Öffnung (3) des ersten Beutels (2) aus erstreckt,
- einen Schritt des Befestigens des Verschlussorgans (4) an dem ersten Beutel (2) auf dichte Weise, mit Hilfe eines Befestigungselements (7) , welches angepasst ist, einen ausreichenden Druck auf den Kragen (5) auszuüben, um ihn zwischen dem Verschlussorgan (4) und dem Befestigungselement (7) einzuklemmen.

18. Verfahren nach Anspruch 17, **dadurch gekennzeichnet, dass** der Schritt des Befestigens des verschlussorgans (4) an dem zweiten Beutel (20) durch Verschweißen oder Verkleben erfolgt.

19. Verfahren nach Anspruch 17 oder 18, **dadurch gekennzeichnet, dass** das Verschlussorgan (4) einen Rand (4B) aufweist, und dadurch, dass das Verfahren einen Unterschritt umfasst, in welchem der Rand (4H) mit der Umrandung des Lochs (21) verschweißt oder verklebt wird.

20. Verfahren nach einem der Ansprüche 17 bis 19, **dadurch gekennzeichnet, dass** es einen Schritt (b) des Umkehrens des Kragens (5) umfasst, derart, dass der Letztere sich im Inneren des ersten Beutels (2) befindet.
